Europäisches Patentamt

European Patent Office

Office européen des brevets

(11) Publication number: **0 255 286**
**A2**

(12) # EUROPEAN PATENT APPLICATION

(21) Application number: **87306480.2**

(22) Date of filing: **22.07.87**

(51) Int. Cl.⁴: **A61K 6/10**

(30) Priority: **28.07.86 US 890049**

(43) Date of publication of application:
**03.02.88 Bulletin 88/05**

(84) Designated Contracting States:
**CH DE FR GB IT LI SE**

(71) Applicant: **Kerr Manufacturing Company (a Delaware corporation)**
**28200 Wick Road Box 455**
**Romulus, Michigan 48174(US)**

(72) Inventor: **Madhavan, Narayanan**
**28925 E.King William**
**Farmington Hills Michigan 48018(US)**
Inventor: **Groh, Carole Landry**
**160 South Evangeline**
**Dearborn Heights Michigan 48125(US)**
Inventor: **Probst, Robert Louis**
**1901 Mershon Drive**
**Ann Arbor Michigan 48103(US)**

(74) Representative: **Oliver, Roy Edward et al**
**POLLAK MERCER & TENCH High Holborn**
**House 52-54 High Holborn**
**London WC1V 6RY(GB)**

(54) **Visible light-cured impression material.**

(57) A dental impression material comprises a pre-polymer formed by reacting a polyol or polyester polyol, a diisocyante, and a hydroxyacrylate or methacrylate, along with a metal catalyst, plasticizer, photosensitizer, reducing agent, filler and other additives. The resultant polymer portion is a long-chain aliphatic polyester-polyurethane molecule, which when end-capped with hydroxyacrylate moieties, is photo-polymerizable to yield a tear-resistant highly flexible medium-durometer elastomer, suitable for use as a dental impression material.

EP 0 255 286 A2

## VISIBLE LIGHT-CURED IMPRESSION MATERIAL

This invention relates to elastomeric dental impression materials. Dental elastomers used as impression materials may be generally classified as being of the polysulphide, condensation silicone, polyvinylsiloxane or polyether types. These materials are normally used as two-paste systems, consisting of a catalyst paste and a base paste. In use, these pastes are mixed in the manufacturer's recommended proportions. The mixing time is generally about 30-45 seconds. The physical properties of these materials, such as flexibility, permanent deformation, compressive set, tear strength and even shrinkage, depend upon the accuracy of the mixing ratio and how well the pastes are mixed within the specified time period. The process of mixing initiates a chemical reaction, which results in the formation of an elastic rubber, the physical properties and dimensional change of which depend upon the nature of the polymerisation reaction. The mixing also results in the incorporation of entrapped air. These air bubbles, together with those already in the material due of the manufacturing process, reduce the accuracy and readability of the impression.

There is therefore a need, which is supplied by the present invention, for the provision of a bubble-free single-component paste for dental impression taking. The invention also provides a low-shrinkage impression paste for obtaining accurate impressions. The invention additionally provides an impression material which can be cured by exposure to visible light of the appropriate wavelength, namely in the range from 400-600 nanometres.

The present invention is directed to a one-component elastomeric dental impression material which can be cured by exposure to radiation of suitable wavelengths. This material is based on urethane acrylates.

More specifically, a dental impression material according to this invention comprises a pre-polymer, formed by reacting a polyol or polyester polyol, a diisocyanate and a hydroxyacrylate or methacrylate, together with, typically but optionally, a metal catalyst, plasticiser, photosensitiser, reducing agent, filler and other additives. The important characteristics of the polymer portion which makes the system work are that it is a long-chain aliphatic polyester-polyurethane molecule, which when end-capped with hydroxyacrylate moieties, is photo-polymerisable to yield a tear-resistant highly-flexible medium-durometer elastomer, which is thus suitable for use as a dental impression material. Impression materials according to this invention meet all the requirements of the American Dental Association specification number 19 for Type 1 materials. The fully-cured material has very low dimensional change, good compression set and strain in compression. The stone models have smooth, bubble-free surfaces.

The impression material of the present invention is preferably composed of the following reactants:

(1) A difunctional polyol or polyester polyol, having a preferred molecular weight of about 1500 to 3000; all suitable polyols, which are essentially aliphatic in nature and of appropriate molecular weight to provide the desired low level of crosslinking in the resultant polymer, may be used. Polyester polyetherdiols are preferred, because the corresponding triols and tetrols significantly increase the crosslink density of the resulting polymer and produce elastomers which are too rigid for use as dental impression materials.

(2) A diisocyanate, aliphatic or aromatic in nature, such as 2,4-toluenediisocyanate, 2,6-toluenediisocyanate or mixtures thereof, hexamethylenediisocyanate, isophoronediisocyanate, trimethylhexamethylenediisocyanate, methylenediisocyanate and cyclohexylmethanediisocyanate, all of which are examples of suitable diisocyanates. Isocyanatoethylmethacrylate may also be used in a single step reaction. Hexamethylenediisocyanate is preferred, because of its straight-chain aliphatic nature.

(3) Another reactant used in carrying out the present invention is a low molecular weight hydroxyacrylate or methacrylate, which terminates the polyurethane at each end. Typical materials include hydroxyethylacrylate, hydroxyethylmethacrylate and hydroxypropylmethacrylate.

The polyol to diisocyante to hydroxyacrylate ratio is theoretically about 1:2:2. However, in order to compensate for the various purity levels of the ingredients and to ensure that the final prepolymer contains no free isocyanate groups, when assayed by titration, a slight modification of this theoretical ratio is used.

The reaction between NCO and OH is generally promoted by a metal catalyst or an amine catalyst. Even though a tertiary amine is present in the system, as mentioned later, a small amount of metal catalyst, especially tin in the form of dibutyltindilaurate or stannous octoate, is employed. The urethane reaction between NCO and OH is highly exothermic and the uncatalysed reaction is extremely slow. In the presence of a plasticiser, which typically forms about 35 percent of the formulation, the temperature is well controlled, to prevent possible side reactions, including the polymerisation of the acrylic compound.

Even though other plasticisers may be employed successfully, Santicizer 261 (octyl benzyl phthalate) available from Monsanto Chemical Company, is particularly suitable. This is an alkylbenzyl-phthalate, the alkyl group having 7 to 9 carbon atoms.

Another material which can be used is a high molecular weight anti-oxidant having hindered phenolic groups. This material has no apparent reaction with isocyanates.

A small amount of surfactant, such as Modaflow, is also desirably employed. This clear viscous liquid is a copolymer of ethylacrylate and 2-ethylhexylacrylate.

The isocyanate content of the pre-polymer formed by reacting the polyol, diisocyante and hydroxyacrylate or methacrylate should be zero. In order to achieve this condition, the amounts of reactants should be balanced stoichiometrically. Thus, the sum of the hydroxy equivalents of the polyol and the hydroxyacrylate or methacrylate should be equal to the isocyanate equivalent. A good elastomeric rubber is obtained if the hydroxy equivalent of the acrylyl chain terminator is either equal to or lower than that of the polyol.

A photosensitizer is added to the reaction mix in order to cure it by actinic radiation in the range from 400 to 600 nanometres. Typical photosensitizers include benzophenone, acetophenone, thioxanthen-9-one, 9-fluorenone, anthraquinone, 4'-methoxyacetophenone, diethoxyacetophenone and the diketones, such as biacetyl, 2,3-pentanedione, benzil, 4,4'-methoxybenzil, 4,4'-oxidibenzil and dl-camphroquinone. Camphroquinone and diketones absorb mostly in the visible light spectrum between 400 and 500 nanometres. Formulations with these initiators cure readily with visible radiation.

The dental impression materials also contain a reducing agent which reduces the ketonic photosensitizers when they are in the excited state and accelerates the rate of polymerization. These reducing agents also reduce the surface tackiness of the cured elastomer. These materials comprise organic amines, aliphatic or aromatic, monoamines, or polyamines, primary, secondary or tertiary. The tertiary amines are generally preferred. Suitable tertiary amines are described in US-A-3,759,807. Tertiary amines with additional functional groups can also be employed, such as 4,4'-bis-(dimethylamino)-benzophenone, N-methyldiethanolamine, 4-dimethlyaminobenzoate, dimethylaminobenzaldehyde, di-methylaminoethylmethacrylate and dimethylaminoethylacrylate.

Another important component of the dental impression material is the filler, which provides added strength and increases the accuracy of the impression. Suitable fillers include talc, calcium carbonate, zinc oxide, glass powder, quartz and mixtures thereof. Silica, especially synthetic silica, because of its higher purity, has a refractive index very close to 1.46 and is a preferred material, as its refractive index matches very closely that of the liquid matrix, so as to maximize light energy transmission in order to maximize the curing efficiency of the material, Aerosil R-972, a hydrophobic submicron synthetic amorphous precipitated silica from Degussa, may be used alone or in combination with other coarser types of silica. Quso WR 55, from PQ Corporation, a synthetic amorphous precipitated silica having an average particle size of about 3 to 4 microns, may also be employed. A combination of Aerosil R-972 and Quso is preferred. In general, the concentration of the filler is preferably about 5 to 20% by weight of the impression material.

In order to reduce further the tackiness due to air inhibition, a small amount of low temperature melting wax can be added to the mix and seems to act synergistically with the tertiary amine. This addition results in a dry, smooth and easy-to-read surface. Readability can be further enhanced by colouring the material with a dye or pigment.

The following example illustrates one embodiment of the present invention. The percentages and parts are by weight.

Example 1.

|  |  | Broad Concentration range by Wt % | Specific Formulation |
| --- | --- | --- | --- |
| Polymer | Rucoflex polyesterdiol S-1011-55* | 20 - 50 | 60.000 parts (.06 equiv.) |
|  | Toluene diisocyanate (Aldrich) | 5 - 20 | 8.700 parts (.1 equiv.) |
|  | 2-hydroxypropyl-methacrylate | 5 - 20 | 5.760 parts (.04 equiv.) |
| Plasticizer | Santicizer 261 (Monsanto) | 20 - 50 | 44.500 parts |
| Fillers | Quso 55 (PQ Corporation) | 5 - 20 | 9.000 parts |
|  | Aerosil R972 (Degussa) | 5 - 20 | 2.500 parts |
| Additives | Dibutyltindilaurate (M & T) | .01 - 0.5 | .050 parts |
|  | Irganox 1010 (Ciba-Geigy)** | .01 - 0.5 | .025 parts |
|  | Modaflow (Monsanto)*** (Plus Paraffin Wax and Pigment) | .05 - 0.2 | .120 parts |
| Curing System | dl-Camphroquinone (Aldrich) | .01 - 1.0 | .080 parts |
|  | Dimethylamino-benzaldehyde | .01 - 1.0 | .060 parts |

\* A polymeric 2,2'-oxybis-(ethanol)-hexanedioic acid ester. $(C_6H_{10}O_4C_4H_{10}O_3)$

\*\* Tetrakis-(methylene-(3,5-di-tert-butyl-4-hydroxy-hydrocinnamate))-methane

\*\*\* Ethyl acrylate and 2-ethyl-hexyl-acrylate copolymer

The specific formulation listed above is prepared by the following procedure:

The Santicizer 261 and polyesterdiol are evacuated for three hours at 85°C to free the materials from moisture and assist in minimizing unwanted side reactions. The fillers are heated in an oven at 150°C for two hours prior to use. All other chemicals are employed in the as-received condition, except the paraffin wax, which, when optionally used is melted and deaerated for three hours at 85°C. During evacuation, the vacuum is maintained at at least 1 mm Hg. The plasticizer is placed in a glass flask and the dimethylaminobenzaldehyde is dissolved in it. The Aerosil is then incorporated and the the Quso. The polyesterdiol is then added and mixed thoroughly. The toluenediisocyanate is then added, at which time the mixture is a mobile liquid. The minor ingredients, such as dibutyltindilaurate, Irganox 1010, Modaflow and camphroquinone, are dissolved in the 2-hydroxypropylmethacrylate by heating in an oven at 50°C for half an hour. They are added after cooling to room temperature. The mix is kept in a dark room during and after preparation.

As the R'NCO + OHR → R'NHCOR urethane reaction proceeds, the viscosity of the mix increases and then stabilises when the reaction is completed. At the end of the reaction, the NCO concentration should be zero. The NCO concentration can be monitored either by a titration technique as described in ASTM 1638 or by infrared spectroscopy. It takes approximately five to seven days to complete the urethane reaction. The paste is readily cured by the visible light energy emitted by a 275 watt General Electric sun lamp three inches (7.5 cm) away from the material.

The following example illustrates another embodiment of the present invention.

4

Example 2

Rucoflex S-1011-55 456.0 parts (.456 equiv)
1,6-Hexanediisocyanate 63.8 parts (.760 equiv)
2-Hydroxypropylmethacrylate 43.8 parts (.304 equiv)
Santicizer 261 338.0 parts
Dimethylaminobenzaldehyde 4.6 parts
Aerosil R-972 19.0 parts
Quso 68.4 parts
Dibutyltindilaurate (T-12) 0.4 parts
Irganox 1010 0.2 parts
Modaflow 0.9 parts
Camphroquinone 0.6 parts
Meteor Cobalt Blue
(Harshaw Chemical) 2.0 parts

A one kilogram batch was made using the above formulation. The raw materials were purified as in Example 1. The dimethylaminobenzaldehyde was dissolved in the Santicizer 261 and the Cobalt blue pigment thoroughly dispersed. After incorporating the Aerosil and Quso, the paste was roll-milled twice. The milled material was placed in a one gallon chrome-plated mixing bowl of a Ross vacuum mixer with planetary blades. Corrections were made for material loss due to roller milling. The subsequent additions are made in this bowl and the photoinitiator was added in a dark room, where radiation capable of curing the material is completely eliminated. Mixing and evacuation were begun and evacuation continued for only five minutes, once the vacuum reached 28 inches. The mix frothed under high vacuum and continued to froth until the vacuum was released. The liquid was transferred to a light-proof metal container in a dark room.

After seven days the reaction between the OH and NCO is completed, as denoted by zero NCO content. The paste is placed in a "4-In-One Tray" (Kerr/Sybron) made of polystyrene and an impression made using a typodont. The light source used is the same as described in Example 1 and the exposure time is five minutes.

The following tests were made according to the ADA specifications, with slight modifications in sample preparation. Consistency of this single paste at 20°C is 42 mm (ADA 4.3.4.). A transparent cylindrical "Plexiglass" mould, 12.5 mm inside diameter and 20 mm high, was filled with the material and the ends flattened with 1 mm thick glass plates. The sample was cured by exposing the ends to the above-mentioned light three inches (7.5 cm) away from the light source. The temperature rise on the material was minimized by a fan placed alongside the sample. The results are shown below:

| Total Cure Time: | 4 Minutes (2 min. each end) | 5 Minutes (2 1/2 min. each end) |
|---|---|---|
| Compression Set % | 0.2 | 0.1 |
| Strain in Compression % | 3.8 | 3.8 |
| Flow | – | 0.2 |
| 24-Hour Dimensional Change % | – | 0.15 |
| Detail Reproduction 0.020 mm | – | Pass |
| Compatibility with Gypsum (Vel-Mix) 0.020 mm | – | Pass |
| Compatibility with Silver Plating Solution | – | Pass |
| 7 day 60°C Deterioration | | Pass |

Details of test procedure are given in the Journal of the American Dental Association, Volume 94, April 1977. The following Example illustrates a further embodiment of the present invention.

Example 3

Santicizer 261 338.00 parts
FD & C Blue 1* .25 parts
Dimethylaminobenzaldehyde 4.60 parts
Paraffin Wax 20.00 parts
Aerosil 19.00 parts
Quso 55 68.40 parts
Rucoflexpolyesterdiol S-1011-55 456.00 parts (.456 equiv.)
1,6 hexamethylenediisocyanate 63.80 parts (.760 equiv.)
2-hydroxypropylmethacrylate 42.80 parts (.304 equiv.)
Modaflow 0.90 parts
Irganox 1010 0.20 parts
Dibutyltindilaurate 0.40 parts
Camphorquinone 1.00 parts
* Disodium salt of ethyl-(4-(p-(ethyl-(m-sulphobenzyl)-amino)-oc-(o-sulphophenyl)-benzylidene)-2,5-cyclohexadien-1-ylidene)-m-sulphobenzyl)-ammonium hydroxide.

The preparation of the above formulation is made in the same manner as in the previous Example. The paraffin wax was melted and added to the plasticizer and whipped prior to the addition of fillers. Adjustments in the filler/plasticizer ratio are used to develop a multiviscosity system. That is, a higher ratio is used to produce a heavy bodied viscosity material, while a lower ratio is used to produce a light bodied viscosity material. Therefore, a variety of materials can be developed to suit a variety of dental applications and techniques.

**Claims**

1. A photopolymerizable elastomeric impression material, characterized by comprising a one-component formulation which includes:
   (a) a pre-polymer formed by reacting a polyol or polyester polyol, a diisocyanate and a hydroxyacrylate or methacrylate;
   (b) a metal catalyst;
   (c) a plasticizer;
   (d) a photosensitizer:
   (e) a reducing agent;
   (f) a filler; and
   (g) an antioxidant, a surfactant, a low-melting wax and a dye or pigment.
2. An impression material according to claim 1, comprising in weight per cent:
20-65% of the pre-polymer,
0.01-1.0% of the metal catalyst,
20-50% of the plasticizer,
0.1-1.0% of the photosensitizer,
0.1-1.0% of the reducing agent, and
5-20% of the filler.
3. An impression material according to claim 2, which further includes, in weight per cent:
0.01-1.0% of the antioxidant
0.05-2.0% of the surfactant
0.1-4.0% of the wax, and
0.01-2.0% of the dye or pigment.
4. An impression material according to claim 1, 2 or 3, comprising:

| | Weight Per cent |
|---|---|
| Polyesterdiol | 20-50 |
| Hexamethylene-diisocyanate | 5-20 |
| Hydroxypropylmethacrylate | 5-20 |
| Alkylbenzylphthalate | 20-50 |
| Dimethylaminobenzaldehyde | .01-1.0 |
| Silica | 5-20 |
| Camphorquinone | .01-1.0 |

5. An impression material according to claim 4, wherein the silica is a synthetic submicron silica.

6. An impression material according to claim 4 or 5, wherein the silica is a synthetic micronized silica.

7. An impression material according to claim 4, wherein the silica is a combination of synthetic submicron and micronized silica.

8. An impression material according to any of claims 4 to 7, which contains an antioxidant in a concentration in the range from 0.01-1.0 weight percent.

9. An impression material according to any of claims 4 to 8, which contains a surfactant.

10. An impression material according to any of claims 4 to 9, which contains a low-melting wax in a concentration in the range from 0.1-4.0 weight percent.

11. An impression material according to any of claims 4 to 10, which contains a dye or pigment in a concentration in the range from 0.01-2.0 weight percent.

12. An impression material according to any preceding claim, wherein the filler/plasticizer ratio is adjusted to provide a multiviscosity system.

13. A method of manufacture of a dental impression, which comprises placing an impression material according to any preceding claim in a light-transmitting dental impression tray, properly seating the tray in a patient's mouth and exposing the tray to visible light for 5 minutes or less to cure the impression material.

14. A dental impression, characterised in that it is made by placing an impression material according to any of claims 1 to 12, in a light-transmitting dental impression tray, properly seating the tray in a patient's mouth and exposing the tray to visible light for 5 minutes or less to cure the impression material.